(19) Europäisches Patentamt
European Patent Office
Office européen des brevets

(11) Veröffentlichungsnummer: **0 180 547 A2**

(12) # EUROPÄISCHE PATENTANMELDUNG

(21) Anmeldenummer: **85810486.2**

(22) Anmeldetag: **25.10.85**

(51) Int. Cl.[4]: **C 07 D 273/04**, A 01 N 43/88 // C07C119/048

(30) Priorität: **31.10.84 CH 5208/84**

(43) Veröffentlichungstag der Anmeldung: **07.05.86 Patentblatt 86/19**

(84) Benannte Vertragsstaaten: **BE CH DE FR GB IT LI NL**

(71) Anmelder: **CIBA-GEIGY AG, Klybeckstrasse 141, CH-4002 Basel (CH)**

(72) Erfinder: **Böger, Manfred, Wilhelm Glockstrasse 14, D-7858 Weil am Rhein 5 (DE)**
Erfinder: **Drabek, Jozef, Dr., Benkenstrasse 12, CH-4104 Oberwil (CH)**

(54) **Oxadiazine und ihre Herstellung sowie diese enthaltende Schädlingsbekämpfungsmittel.**

(57) Neue N-Phenyl-1,3,5-oxadiazin-2,4-dione der Formel I

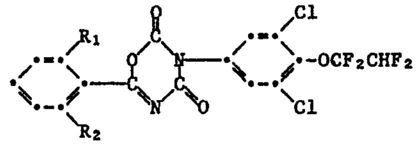

(I)

worin

$R_1$ Fluor oder Chlor und

$R_2$ Wasserstoff, Fluor oder Chlor

bedeuten, ihre Herstellung, ihre Verwendung in der Schädlingsbekämpfung und Schädlingsbekämpfungsmittel, welche diese Oxadiazine als Wirkstoff enthalten, werden offenbart. Bevorzugtes Anwendungsgebiet ist die Bekämpfung von Schädlingen an Tieren und Pflanzen.

EP 0 180 547 A2

0180547

CIBA-GEIGY AG
Basel (Schweiz)

5-15130/+

# Oxadiazine und ihre Herstellung sowie diese enthaltende Schädlingsbekämpfungsmittel

Die vorliegende Erfindung betrifft neue N-Phenyl-1,3,5-oxadiazin-2,4-dione, ihre Herstellung, ihre Verwendung in der Schädlingsbekämpfung sowie Schädlingsbekämpfungsmittel, welche diese Oxadiazine enthalten.

Aus der DE-OS 29 05 687 sind solche N-Phenyl-1,3,5-oxadiazin-2,4-dione als insektizide Wirkstoffe bekannt, in denen das N-Phenyl nicht gleichzeitig in 3,5-Stellung durch Cl und in 4-Stellung durch 1,1,2,2-Tetrafluoräthoxy substituiert ist.

Die erfindungsgemässen Oxadiazine entsprechen der Formel I

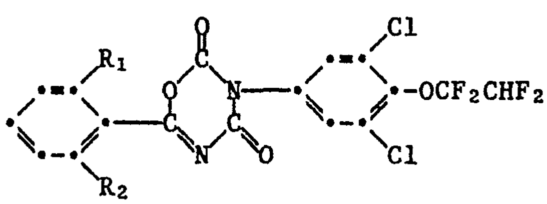

(I),

worin

$R_1$ Fluor oder Chlor und

$R_2$ Wasserstoff, Fluor oder Chlor

bedeuten.

Dabei sind diejenigen Verbindungen der Formel I bevorzugt, in denen $R_1$ und $R_2$ gleich sind und Fluor oder Chlor bedeuten.

0180547

Als Beispiel einer Verbindung der Formel I sei erwähnt:

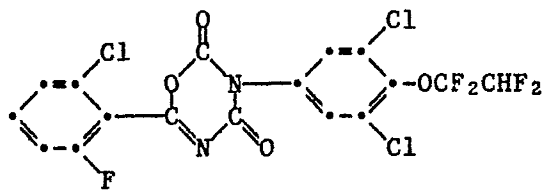

Die erfindungsgemässen Verbindungen können nach an sich bekannten Verfahren hergestellt werden. Solche Verfahren sind u.a. in den DE- OS 27 32 115 und 29 05 687 beschrieben. So können die Verbindungen der Formel I z.B. erhalten werden, indem man ein Benzoylisocyanat der Formel II

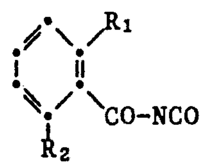

(II)

mit dem Isocyanat der Formel III

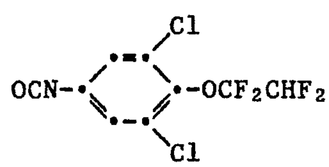

(III)

umsetzt. Zur Durchführung des Verfahrens erhitzt man die beiden Reaktionskomponenten gegebenenfalls in Gegenwart eines Lösungs- oder Verdünnungsmittels während 30 Minuten bis 30 Stunden auf eine Temperatur im Bereich von 50 bis 150°C, vorzugsweise während 5 bis 15 Stunden auf eine Temperatur im Bereich von 80 bis 120°C. Als Lösungs- oder Verdünnungsmittel eignen sich vor allem polare aprotische Lösungsmittel wie z.B. Dimethylsulfoxid, Dimethylformamid oder N,N-Dimethylacetamid.

Die erfindungsgemässen Verbindungen können aber auch nach anderen Methoden hergestellt werden, z.B. indem man

a) ein Halogencarbonylbenzamid der Formel IV

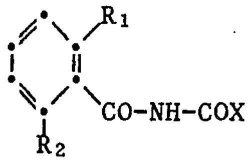

(IV)

mit dem Isocyanat der Formel III, oder

b) ein Benzoylisocyanat der Formel II mit einem Carbaminsäureester der Formel V

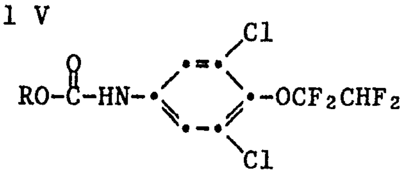

(V)

kondensiert. In den Formeln II, IV und V haben die Reste $R_1$ und $R_2$ die für Formel I angegebene Bedeutung, während X für Halogen, vorzugsweise Chlor, und R für einen Alkylrest, vorzugsweise einen niederen Alkylrest mit 1 bis 4 Kohlenstoffatomen, stehen.

Die Verbindungen der Formeln II bis V sind bekannt oder können nach bekannten Methoden hergestellt werden. So können die Benzoylisocyanate der Formel II durch Umsetzung der entsprechend substituierten Benzamide mit Oxalylchlorid hergestellt und das Isocyanat der Formel III aus dem entsprechend substituierten Anilin durch Umsetzung mit Phosgen gewonnen werden.

Die erfindungsgemässen Verbindungen weisen überraschenderweise gegenüber den aus der DE-OS 29 05 687 bekannten Verbindungen eine überlegene Wirkung gegen Heliothis virescens und Spodoptera littoralis auf. Ganz allgemein sind sie bei günstiger Warmblüter- und Pflanzenverträglichkeit wertvolle Wirkstoffe in der Schädlingsbekämpfung.
So eignen sich die Verbindungen der Formel I z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und

Arachniden der Ordnung Acarina,wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formel I von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus- und Nutztieren, kommen vor allem Ektoparasiten, wie z.B. Milben und Zecken und Dipteren, wie z.B. Lucilia sericata in Betracht. Die gute Wirkung der erfindungsgemässen Verbindungen entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als solche Zusätze kommen z.B. organische Phosphorverbindungen, Nitrophenole und Derivate, Formamidine, Harnstoffe, Carbamate, Pyrethroide, chlorierte Kohlenwasserstoffe und Bacillus thuringiensis-Präparate in Betracht.

Mit besonderem Vorteil kann man die Verbindungen der Formel I auch mit Substanzen kombinieren, welche einen pestizid verstärkenden Effekt ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten

Emulsionen, Spritzpulvern, löslichen Pulvern, Stäubemitteln, Granulaten oder auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, d.h. die einen Wirkstoff der Formel I bzw. Kombinationen dieser Wirkstoffe mit andern Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden.

Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand, in Frage. Darüberhinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombination dieser Wirkstoffe mit anderen Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat, in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl -oder niedrige Hydroxyalkylreste aufweisen. Die Salze liegen vorzugsweise

als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in den folgenden Publikationen beschrieben:

"Mc Cutcheon's Detergents and Emulsifiers Annual" MC Publishing Corp., Ridgewood, New Jersey, 1981; Dr. Helmut Stache "Tensid-Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit anderen Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze, wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung

1.1: 3,5-Dichlor-4-(1,1,2,2-tetrafluoräthoxy)-phenylisocyanat

33,4 g 3,5-Dichlor-4-(1,1,2,2-tetrafluoräthoxy)-anilin werden in 130 ml Chlorbenzol gelöst und tropfenweise unter Rühren bei 22°C in eine Lösung bestehend aus 90 g 20 Gew.% Phosgen enthaltendem Toluol, 100 ml Dioxan und 300 ml Chlorbenzol gegeben. Das Reaktionsgemisch wird dann je eine Stunde lang bei Raumtemperatur, 50°C und 80°C nachgerührt. Anschliessend wird am Wasserstrahlvakuum eingeengt und am Hochvakuum destilliert. Man erhält die Titelverbindung der Formel

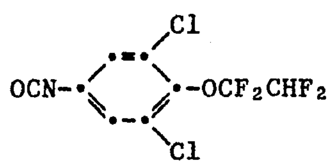

(III)

als farbloses Oel. Kp 81-83°C/2,5 mbar.

1.2: 3-[3,5-Dichlor-4-(1,1,2,2-tetrafluoräthoxy)-phenyl]-6-(2,6-difluorphenyl)-3,4-dihydro-2H-1,3,5-oxadiazin-2,4-dion

Unter Feuchtigkeitsausschluss werden 9,1 g 3,5-Dichlor-4-(1,1,2,2-tetrafluoräthoxy)-phenylisocyanat unter Rühren mit 5,4 g 2,6-Difluorbenzoylisocyanat versetzt. 14 Stunden lang wird die Temperatur auf 120°C gehalten, wobei so lange gerührt wird, bis das Reaktionsgemisch zu erstarren beginnt. Wenn sich der entstandene Kristallbrei auf Raumtemperatur abgekühlt hat, wird er mit Hexan zerrieben und anschliessend abgenutscht. Der Rückstand wird aus Toluol umkristallisiert. Man erhält die Titelverbindung der Formel

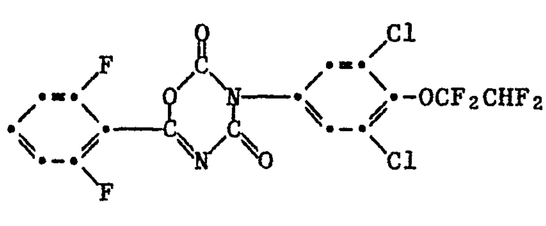

als weisses Pulver mit einem Smp. von 170-172°C (Verbindung Nr. 1.2.1)

Auf analoge Weise werden die folgenden Verbindungen hergestellt:

| Verbindung Nr. | $R_1$ | $R_2$ | Smp. °C |
|---|---|---|---|
| 1.2.2 | Cl | Cl | 182-184 |
| 1.2.3 | Cl | H | 167-169 |
| 1.2.4 | F | H | 166-168 |

Beispiel 2: Formulierungen von Wirkstoffen der Formel I gemäss Herstellungsbeispiel 1

(% = Gewichtsprozent)

| 2.1 Emulsions-Konzentrate | a) | b) | c) | d) |
|---|---|---|---|---|
| Wirkstoff gemäss dem Herstellungsbeispiel 1 | 10 % | 25 % | 40 % | 50 % |
| Ca-Dodecylbenzolsulfonat | - | 5 % | 8 % | 6 % |
| Ricinusöl-polyäthylenglykoläther (36 Mol AeO) | - | 5 % | - | - |
| Tributylphenol-polyäthylenglykoläther (30 Mol AeO) | - | - | 12 % | 4 % |
| Ricinusölthioxilat | 25 % | - | - | - |
| Cyclohexanon | - | - | 15 % | 20 % |
| Butanol | 15 % | - | - | - |
| Xylolgemisch | - | 65 % | 25 % | 20 % |
| Essigester | 50 % | - | - | - |

Aus solchen Konzentraten können durch Verdünnen mit Wasser Emulsionen jeder gewünschten Konzentration hergestellt werden.

| 2.2 Lösungen | a) | b) |
|---|---|---|
| Wirkstoff gemäss dem Herstellungsbeispiel 1 | 10 % | 5 % |
| Aethylenglykol-monomethyläther | - | - |
| Polyäthylenglykol (MG 400) | 70 % | - |
| N-Methyl-2-pyrrolidon | 20 % | - |
| Epoxidiertes Kokosnussöl | - | 1 % |

| | | |
|---|---|---|
| Benzin (Siedegrenzen 160-190°C) | - | 94 % |

Die Lösungen sind zur Anwendung in Form kleinster Tropfen geeignet.

| 2.3 Granulate | a) | b) |
|---|---|---|
| Wirkstoff gemäss dem Herstellungsbeispiel 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) | c) | d) |
|---|---|---|---|---|
| Wirkstoff gemäss dem Herstellungsbeispiel 1 | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff erhält man gebrauchsfertige Stäubemittel.

| 2.5 Spritzpulver | a) | b) | c) |
|---|---|---|---|
| Wirkstoff gemäss dem Herstellungsbeispiel 1 | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

2.6 Extruder Granulat

| | |
|---|---|
| Wirkstoff gemäss dem Herstellungsbeispiel 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.7 Umhüllungs-Granulat

| | |
|---|---|
| Wirkstoff gemäss dem Herstellungsbeispiel 1 | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

2.8 Suspensions-Konzentrat

| | |
|---|---|
| Wirkstoff gemäss dem Herstellungsbeispiel 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |

| | |
|---|---|
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologische Prüfungen

3.1 Wirkung gegen Musca domestica

Je 50 g frisch zubereitetes CSMA-Nährsubstrat für Maden werden in einen Becher eingewogen. Von einer 1 gew.%igen acetonischen Lösung des Wirkstoffes werden 5 ml auf das im Becher befindliche Nährsubstrat pipettiert. Nach dem Durchmischen des Substrates lässt man das Aceton mindestens 20 Stunden lang verdampfen.

Dann werden 25 eintägige Maden von Musca domestica in den das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in einem mit einem Siebdeckel verschlossenen Gefäss deponiert.

Die ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen gemäss Beispiel 1 zeigen gute Wirkung im obigen Test.

3.2 Wirkung gegen Lucilia sericata

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss Beispiel 1 zeigen in diesem Test gute Wirkung gegen Lucilia sericata.

3.3 Wirkung gegen Aedes aegypti

Auf die Oberfläche von 150 ml Wasser, das sich in einem Behälter befindet, wird so viel einer 0,1 %igen acetonischen Lösung des Wirkstoffes pipettiert, dass eine Konzentration von 12,5 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss Beispiel 1 zeigen gute Wirkung in diesem Test.

3.4 Insektizide Frassgift-Wirkung

Baumwollpflanzen (ca. 20 cm hoch) werden mit einer wässrigen Wirkstoffemulsion (erhalten aus einem 10%igen emulgierbaren Konzentrat) besprüht, wobei die Wirkstoffemulsion 100 ppm der zu prüfenden Verbindung enthält.

Nach dem Antrocknen des Belages werden die Baumwollpflanzen je mit Spodoptera littoralis- und Heliothis virescens-Larven. im dritten larvalen Stadium besetzt. Der Versuch wird bei 24°C und 60 % relativer Luftfeuchtigkeit durchgeführt. In Abständen von jeweils 24 Stunden werden Mortalität sowie Entwicklungs- und Häutungsstörungen der angesetzten Larven bestimmt.

Verbindungen gemäss Beispiel 1 zeigen gute Wirkung in diesem Test.

3.5 Wirkung auf Spodoptera littoralis und Heliothis virescens (Larven und Eier)

Es werden drei in Töpfen gezogene Baumwollpflanzen von ca. 15-20 cm Höhe mit einer sprühfähigen flüssigen Zubereitung des zu prüfenden Wirkstoffes behandelt. Nach Antrocknen des Sprühbelages werden die eingetopften Pflanzen in ein Blechgefäss von etwa 20 Litern Inhalt gestellt, das mit einer Glasplatte abgedeckt wird. Die Feuchtigkeit im Inneren des abgedeckten Gefässes wird so reguliert, dass sich kein Kondenswasser bildet. Direktes, auf die Pflanzen fallendes Licht wird vermieden. Dann werden die drei Pflanzen infestiert, und zwar insgesamt mit:

a) 50 Larven von Spodoptera littoralis oder Heliothis virescens des ersten larvalen Stadiums;
b) 20 Larven von Spodoptera littoralis oder Heliothis virescens des dritten larvalen Stadiums;
c) zwei Eispiegeln von Spodoptera littoralis oder Heliothis virescens (dazu werden je 2 Blätter einer Pflanze in einem beidseitig mit Gaze verschlossenen Plexiglaszylinder eingeschlossen); zwei Eispiegel von Spodoptera oder ein Teil eines Baumwollblattes mit darauf abgelegten Eiern von Heliothis werden zu den eingeschlossenen Blättern gegeben.

Nach 4 und 5 Tagen erfolgt die Auswertung gegenüber unbehandelten Kontrollen unter Berücksichtigung folgender Kriterien:

a) Anzahl der noch lebenden Larven,
b) larvale Entwicklungs- und Häutungshemmung,
c) Frassschaden (Schabfrass und Lochfrass),
d) Schlupfrate (Anzahl der aus den Eiern geschlüpften Larven).

Verbindungen gemäss Beispiel 1 zeigen in einer Konzentration von 400 ppm gute Gesamt-Wirksamkeit in obigem Test.

3.6 Ovizide Wirkung auf Spodoptera littoralis

Auf Filterpapier abgelegte Eier von Spodoptera littoralis werden aus dem Papier ausgeschnitten und in eine 0,05 gew.%ige Lösung des Wirkstoffes in einem Aceton/Wasser-Gemisch (1:1) getaucht. Die so behandelten Eiablagen werden dann aus diesem Gemisch herausgenommen und bei 28°C und 60 % relativer Luftfeuchtigkeit in Kunststoffschalen deponiert.

Nach 5 Tagen wird die Schlupfrate, d.h. die Anzahl Larven, die sich aus den behandelten Eiern entwickelt haben, bestimmt.

Verbindungen gemäss Beispiel 1 zeigen gute Wirkung im obigen Test.

3.7 Wirkung auf Laspeyresia pomonella (Eier)

Abgelegte Eier von Laspeyresia pomonella, die nicht älter als 24 Stunden sind, werden auf Filterpapier für 1 Minute in eine acetonisch-wässrige Lösung, enthaltend 400 ppm des zu prüfenden Wirkstoffes, eingetaucht. Nach dem Eintrocknen der Lösung wird das Filterpapier mit den Eiern in einer Petrischale ausgelegt und bei einer Temperatur von 28°C belassen. Nach 6 Tagen wird der prozentuale Schlupf aus den behandelten Eiern bewertet.

Verbindungen gemäss Beispiel 1 zeigen gute Wirkung in obigem Test.

3.8 Reproduktions-Beeinflussung von Anthonomus grandis

Adulte Anthonomus grandis, die nach dem Schlupf nicht älter als 24 Stunden sind, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 0,1 Gew.% des zu prüfenden Wirkstoffes, eingetaucht.

Wenn die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann

in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraumes von etwa vier Wochen überprüft. Die Bonitierung erfolgt anhand der Verminderung der Anzahl abgelegter Eier und der daraus geschlüpften Larven im Vergleich zu unbehandelten Kontrollen.

Verbindungen gemäss Beispiel 1 zeigen eine gute reproduktionsreduzierende Wirkung im obigen Test.

3.9 Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen gemäss Beispiel 1 zeigen gute Wirkung in diesem Test.

0180547

Patentansprüche

1. Verbindungen der Formel I

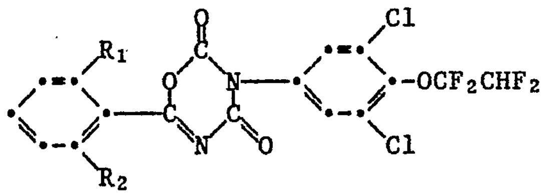

(I),

worin

$R_1$ Fluor oder Chlor und

$R_2$ Wasserstoff, Fluor oder Chlor

bedeuten.

2. Verbindungen der Formel I gemäss Anspruch 1, worin $R_1$ und $R_2$ gleich sind und Fluor oder Chlor bedeuten.

3. Verbindung gemäss Anspruch 2 der Formel

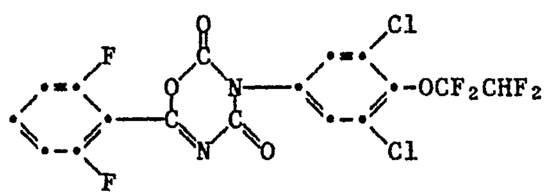

.

4. Verbindung gemäss Anspruch 2 der Formel

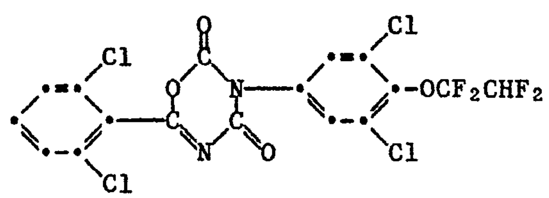

.

5. Verbindung gemäss Anspruch 1 der Formel

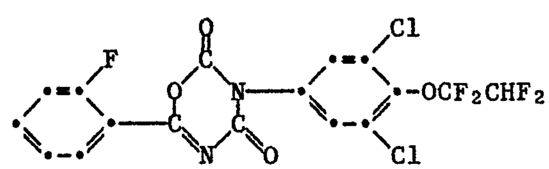

.

6. Verbindung gemäss Anspruch 1 der Formel

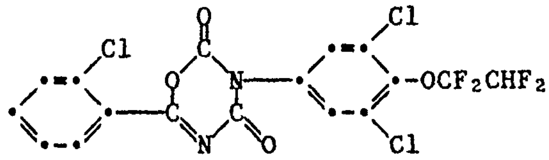

7. Verfahren zur Herstellung einer Verbindung der Formel I

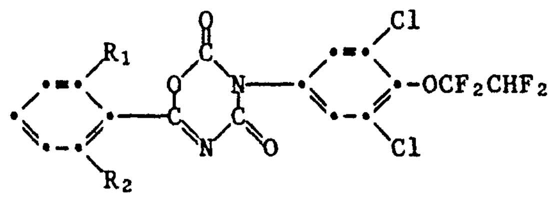

(I),

worin

$R_1$ Fluor oder Chlor und

$R_2$ Wasserstoff, Fluor oder Chlor

bedeuten, dadurch gekennzeichnet, dass man ein Benzoylisocyanat der Formel II

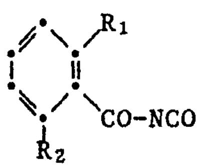

(II)

mit dem Isocyanat der Formel III

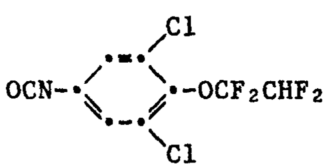

(III)

in bekannter Weise umsetzt, wobei in der Formel II die Symbole $R_1$ und $R_2$ die für Formel I angegebene Bedeutung haben.

8. Verfahren gemäss Anspruch 7, dadurch gekennzeichnet, dass in Formel I $R_1$ und $R_2$ gleich sind und für Fluor oder Chlor stehen.

9. Schädlingsbekämpfungsmittel, welches als aktive Komponente eine Verbindung der Formel I

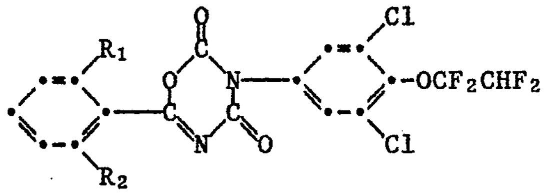

(I),

enthält, worin

$R_1$ Fluor oder Chlor und

$R_2$ Wasserstoff, Fluor oder Chlor

bedeuten, zusammen mit geeigneten Trägern und/oder Zuschlagstoffen.

10. Verwendung einer Verbindung der Formel I

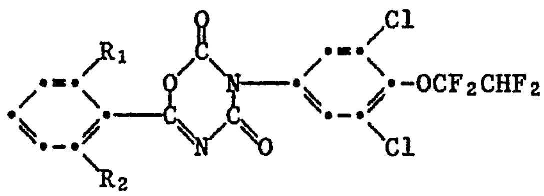

(I),

worin

$R_1$ Fluor oder Chlor und

$R_2$ Wasserstoff, Fluor oder Chlor

bedeuten, zur Bekämpfung von Schädlingen an Tieren und Pflanzen.

11. Verwendung gemäss Anspruch 10 zur Bekämpfung von Arthropoden, insbesondere pflanzenschädigenden Insekten und Arachniden.

12. Verfahren zum Bekämpfen von Schädlingen an Tieren und Pflanzen, dadurch gekennzeichnet, dass man die Schädlinge in ihren verschiedenen Entwicklungsstadien mit einer Verbindung der Formel I

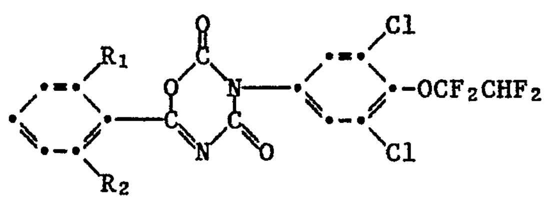

(I),

worin

$R_1$ Fluor oder Chlor und

$R_2$ Wasserstoff, Fluor oder Chlor

bedeuten, in Kontakt bringt.

FO 7.5/BY/cs*